(19) Europäisches Patentamt
European Patent Office
Office européen des brevets

(11) **EP 1 942 344 B1**

(12) **EUROPÄISCHE PATENTSCHRIFT**

(45) Veröffentlichungstag und Bekanntmachung des Hinweises auf die Patenterteilung:
**14.08.2013   Patentblatt 2013/33**

(51) Int Cl.:
*G01N 27/04* (2006.01)      *G01N 33/38* (2006.01)

(21) Anmeldenummer: **07024946.1**

(22) Anmeldetag: **21.12.2007**

(54) **Verfahren zur Untersuchung eines Materialverhaltens in einem Baustoff**

Method for inspecting the behaviour of a material in a construction material

Procédé destiné à l'examen du comportement d'un matériau dans un matériau de construction

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **08.01.2007   DE 102007002007**

(43) Veröffentlichungstag der Anmeldung:
**09.07.2008   Patentblatt 2008/28**

(73) Patentinhaber: **Bilfinger SE**
**68165 Mannheim (DE)**

(72) Erfinder:
- **Schmidt, Detlef, Dr.**
  **04207 Leipzig (DE)**
- **Slowik, Volker, Prof. Dr.-Ing.**
  **04228 Leipzig (DE)**
- **Schmidt, Markus**
  **04275 Leipzig (DE)**
- **Fritzsch, Roberto**
  **04275 Leipzig (DE)**

(74) Vertreter: **Ullrich & Naumann**
**Patent- und Rechtsanwälte**
**Schneidmühlstrasse 21**
**69115 Heidelberg (DE)**

(56) Entgegenhaltungen:
**US-A1- 2004 153 270**

- **HAMMER T A ET AL: "Influence of entrained air voids on pore water pressure and volume change of concrete before and during setting" MATERIALS AND STRUCTURES, KLUWER ACADEMIC PUBLISHERS, DO, Bd. 39, Nr. 9, 2. August 2006 (2006-08-02), Seiten 801-808, XP019456708 ISSN: 1871-6873**
- **MCCARTER W J ET AL: "Field monitoring of electrical conductivity of cover-zone concrete" CEMENT AND CONCRETE COMPOSITES, ELSEVIER APPLIED SCIENCE, BARKING, GB, Bd. 27, Nr. 7-8, August 2005 (2005-08), Seiten 809-817, XP004952528 ISSN: 0958-9465**
- **KEWEN L ET AL: "Determination of Capillary Pressure Function from Resistivity Data" TRANSPORT IN POROUS MEDIA, KLUWER ACADEMIC PUBLISHERS, DO, Bd. 67, Nr. 1, 29. November 2006 (2006-11-29), Seiten 1-15, XP019484821 ISSN: 1573-1634**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft ein Verfahren zur Untersuchung eines Materialverhaltens in einem Baustoff, insbesondere in Beton. Des Weiteren wird eine Vorrichtung zur Untersuchung eines Materialverhaltens in einem Baustoff, insbesondere in Beton und insbesondere zur Durchführung des obigen Verfahrens, beschrieben.

Verfahren und Vorrichtungen der eingangs genannten Art, mit denen Untersuchungen eines Materialverhaltens in einem Baustoff durchgeführt werden, sind aus der Praxis bekannt. Dabei werden diese im Folgenden beispielhaft anhand des Baustoffs Beton beschrieben.

An frisch betonierten Betonbauteilen kann es infolge ungünstiger klimatischer Bedingungen - Sonneneinstrahlung, Wind und/oder geringe relative Luftfeuchte - mit hohen Verdunstungsraten verstärkt zu plastischen Schwindvorgängen - Kapillar-oder Frühschwinden - und dadurch bedingter Rissbildung kommen. Die entstehenden Risse sind in der Regel tiefgehend und schädigen das Bauwerk.

Die Ursache für das plastische Schwinden ist ein sich im Baustoff einstellender kapillarer Unterdruck. Er entsteht infolge einer durch Adhäsionskräfte und die Oberflächenspannung des Wassers bedingten Meniskenbildung zwischen den einzelnen Partikeln an der Oberfläche. Der Druckaufbau erfolgt jedoch erst, nachdem die Anfangssetzung des Betons sowie das damit einhergehende Bluten abgeklungen sind und der dabei entstandene Wasserfilm auf der Oberfläche verdunstet ist.

Der Kapillardruck bewirkt durch die Haftkraft des Wassers eine Kontraktion des noch plastischen Baustoffs. Die Partikelabstände und damit das Gesamtvolumen des Bauteils verringern sich. In diesem Stadium der Gefügeentwicklung sind alle Porenräume wassergefüllt und miteinander verbunden. Es findet ein Druckausgleich zwischen den Poren statt.

Das plastische Schwinden ist ein physikalischer Vorgang. Die Festigkeitsentwicklung infolge Hydratation spielt zu diesem frühen Zeitpunkt noch eine untergeordnete Rolle. Unterschreitet der Kapillardruck die vom Gefüge, insbesondere der Partikelgrößenverteilung, abhängige Größe nach der folgenden Gleichung (1), so beginnt der Wasserspiegel in den größeren Poren schnell abzusinken.

$$p \le -\frac{2 \cdot \gamma}{r} \qquad\qquad (1)$$

$p$   Kapillardruck in [N/m$^2$] oder [Pa]
$\gamma$   Oberflächenspannung der Porenlösung [N/m$^2$]
$r$   Meniskenradius in [m]

Dabei wird das System instabil und es kommt zu einer schlagartigen Umverteilung des vorhandenen Wassers, bei der das innere Gleichgewicht wieder hergesellt wird. Hierbei tritt Luft in das System ein. Die Partikelzwischenräume - Kapillarporen - sind nun nicht mehr vollständig mit Wasser gefüllt, jedoch bleiben die einzelnen Partikel weiterhin durch sogenannte Wassermanschetten verbunden, über welche die Haftkräfte des Wassers wirken.

Der Zeitpunkt der Porenbelüftung wird als Lufteintrittspunkt bezeichnet. Nach der Porenbelüftung ist die Rissgefährdung am größten, da die belüfteten Poren Schwachstellen im Gefüge darstellen. Wird die zu dem Zeitpunkt noch geringe Zugfestigkeit des Baustoffs lokal überschritten, kommt es zur Rissbilung.

Von den beschriebenen Vorgängen sind besonders Betone mit hohem Zementanteil sowie solche, die Betonzusatzstoffe mit kleinen Partikelgrößen - SFA, Silikastaub - enthalten, betroffen. In diesen Materialien, so zum Beispiel in feinkornreichen Hochleistungsbetonen, können sich kleinere Meniskenradien mit damit verbundenen hohen Kapillardrücken einstellen. Der Einsatz von Zementen mit spätem Erstarrungsbeginn begünstigt ebenfalls das kapillare Schwinden. Hier kann bei ungünstigen Bedingungen über einen längeren Zeitraum Wasser aus dem noch plastischen Beton verdunsten, bevor eine nennenswerte Anfangsfestigkeit erreicht wird.

Als Hilfsmittel für die Beurteilung des Wasserverlustes im frühen Alter und das damit einhergehende plastische Schwinden stehen das Nomogramm nach Menzel sowie das sogenannte Curing-Meter zur Verfügung. Ersteres setzt eine Messung bzw. Abschätzung von Beton- und Lufttemperatur, Windgeschwindigkeit sowie der relativen Luftfeuchte voraus. Beim Curing-Meter verdunstet Wasser aus einer definierten Geometrie. Die Verdunstungsbedingungen entsprechen dabei denen an der Betonoberfläche, so dass Rückschlüsse auf den Wasserverlust aus dem Beton möglich sind.

Nach DIN 1045-3 ist mit einer Nachbehandlung des Betons unmittelbar nach Abschluss des Verdichtens oder der Oberflächenbearbeitung zu beginnen. Mit einer derartigen Nachbehandlung kann der Wasserverlust im Beton infolge Verdunstung reduziert werden. Eine nähere Betrachtung des frühen Schwindens erfolgt in den derzeit gültigen Normenwerken jedoch nicht.

Wann tatsächlich mit der Nachbehandlung begonnen wird, hängt oftmals von den technologischen und örtlichen Gegebenheiten sowie von der Erfahrung der Verantwortlichen auf der Baustelle ab. Oftmals werden Nachbehandlungsmaßnahmen erst eingeleitet, wenn bereits ein Kapillardruckaufbau stattgefunden hat und/oder die Nachbehandlungsmaßnahmen den Druckaufbau nicht mehr unterbinden können. Es besteht die Gefahr, dass es vor oder während der Nachbehandlung zu einer sichtbaren oder einer unsichtbaren Schädigung des Gefüges in Form von Trennrissen oder Mikrorissen kommt. Dies ist besonders bei ungünstiger Witterung mit hohen Verdunstungsraten der Fall. Die zur Verfügung stehenden Hilfsmittel wie das Nomogramm nach Menzel und das Curing-Meter können Anhaltswerte über den Wasserverlust infolge Verdunstung geben. Eine direkte Charakterisierung der Vorgänge im Beton lassen sie jedoch nicht zu.

Gemäß dem Stand der Technik gibt es keine Möglichkeit, die Gefährdung von Betonflächen durch plastische Schwindvorgänge auf direktem Weg zu bestimmen.

Bei dem aus der US 2004/0153270 A1 bekannten Qualitätsüberwachungssystem für Bauwerkstrukturen werden zwar Kapillardruck und elektrische Leitfähigkeit ab Baustoffeinbau von Halbleiterchips gemessen, die der flüssigen Betonpaste hinzugefügt werden und die anormale Messwerte in einem internen Speicher ablegen. Diese Daten werden jedoch erstmals nach dem Aushärten des Betons drahtlos ausgelesen, um nachträglich Informationen über den Aushärtevorgang und damit die Qualität der Bauwerksstruktur zu erhalten, sowie später zur Überwachung des Alterungszustandes des Bauwerks.

Der vorliegenden Erfindung liegt daher die Aufgabe zugrunde, ein Verfahren zur Untersuchung eines Materialverhaltens in einem Baustoff anzugeben, wonach eine besonders hohe Qualität eines mit dem Baustoff hergestellten Bauwerks auf einfache Weise erreichbar ist.

Erfindungsgemäß wird die voranstehende Aufgabe durch ein Verfahren zur Untersuchung eines Materialverhaltens in einem Baustoff, insbesondere in Beton, mit den Merkmalen des Patentanspruchs 1 gelöst.

In erfindungsgemäßer Weise ist erkannt worden, dass durch die Kombination einer Messung des Kapillardrucks und einer Messung der elektrischen Leitfähigkeit im Baustoff die voranstehende Aufgabe auf überraschend einfache Weise gelöst wird. Dabei lässt sich der kapillare Unterdruck in beispielsweise Beton mittels Drucksensoren messen. Im Falle der Porenbelüftung am Messort des Drucksensors bricht der gemessene Druck zusammen. Er entspricht dann etwa dem Atmosphärendruck. Der Zusammenbruch erfolgt gefügebedingt je nach Ort zu verschiedenen Zeiten. Außerdem kann es in einem Zwischenstadium zu einer Inselbildung mit noch vollständig wassergefüllten, d. h. noch nicht belüfteten Bereichen kommen. Liegt der Sensor in einem solchen Bereich, erfolgt der Zusammenbruch später und der Druck steigt zumeist weiter an. Die absolute Höhe des örtlich gemessenen Kapillardrucks kann daher nicht als Kriterium zur Beurteilung der Schwindrissgefährdung genutzt werden.

Die Messung der elektrischen Leitfähigkeit ermöglicht weiterhin eine Charakterisierung von chemischen und physikalischen Vorgängen in beispielsweise Beton. Die Belüftung der Porenräume mit der damit verbundenen veränderten Wasserverteilung in den Poren bewirkt eine signifikante Änderung der Leitfähigkeit des Baustoffs, da der Stromfluss nur noch punktuell über die Wassermanschetten möglich und daher stark reduziert ist.

Durch die Kombination der Messung des Kapillardrucks und der elektrischen Leitfähigkeit ist eine deutlich genauere Identifikation des Lufteintrittspunkts bei der Porenbelüftung möglich, als dies bei einer reinen Kapillardruckmessung möglich ist. Aufwändige Korrekturen der Messwerte in Folge chemischer Vorgänge, beispielsweise Änderung der Ionenkonzentration in der Porenlösung, sowie eine kontinuierliche Temperaturkompensation sind aufgrund des eng begrenzten Zeitraums und der deutlich messbaren plötzlichen Leitfähigkeitsänderung nicht oder nur bedingt erforderlich.

Aufgrund der verbesserten Identifikation des Lufteintrittspunkts können Nachbehandlungen des Baustoffs zur Verminderung von Bauschäden durch plastisches Schwinden zeitlich exakt terminiert durchgeführt werden. Daher ist mit dem erfindungsgemäßen Verfahren eine besonders hohe Qualität eines mit dem Baustoff hergestellten Bauwerks auf einfache Weise erreichbar.

Im Hinblick auf eine sichere Bestimmung des Lufteintrittspunkts bei der Porenbelüftung in beispielsweise Beton könnte der Kapillardruck kontinuierlich oder nach vorgebbaren Zeitabständen oder an vorgebbaren Zeitpunkten innerhalb eines vorgebbaren Zeitintervalls, jeweils ab dem Einbau des Baustoffs, gemessen werden. Dabei ist eine kontinuierliche Messung am günstigsten, um einen genauen zeitlichen Verlauf des Kapillardrucks erfassen zu können. Bei einfacheren Messverfahren könnte eine Messung nach vorgebbaren Zeitabständen oder an vorgebbaren Zeitpunkten erfolgen.

Ebenfalls im Hinblick auf eine sichere Bestimmung des Lufteintrittspunkts könnte die Leitfähigkeit kontinuierlich oder nach vorgebbaren Zeitabständen oder an vorgebbaren Zeitpunkten innerhalb eines vorgebbaren Zeitintervalls gemessen werden. Auch hier ist eine kontinuierliche Messung der Leitfähigkeit am günstigsten.

Im bestmöglichen Fall könnten der Kapillardruck und die Leitfähigkeit gleichzeitig gemessen werden. Dies ermöglicht den direkten Vergleich dieser Parameter zur sicheren Bestimmung des Lufteintrittspunkts bei der Porenbelüftung.

Zur Gewährleistung einer besonders guten messtechnischen Darstellung der Veränderung der Leitfähigkeit mit der Zeit könnte die Leitfähigkeit in unterschiedlichen Tiefen im Baustoff gemessen werden. Eine derartige Messung könnte jeweils an verschiedenen Punkten in unterschiedlichen Tiefen erfolgen. Die Messung in verschiedenen Tiefen ermöglicht die Beobachtung des Verlaufs der Porenbelüftung in der Umgebung eines entsprechenden Messsensors und verbessert somit die Identifikation des Lufteintrittspunkts. In besonders vorteilhafter Weise könnte zur Messung der Leitfähigkeit eine tiefengestaffelte Leitfähigkeitssonde verwendet werden. Eine derartige Sonde könnte aus in verschiedenen Tiefen angeordneten Elektroden bestehen. Mit einer derartigen tiefengestaffelten Leitfähigkeitssonde ist eine tiefengestaffelte Leitfähigkeitsmessung sicher möglich.

Auf besonders einfache Weise könnte der Kapillardruck mittels eines Drucksensors mit einer in den Baustoff eingebrachten oder einbetonierten Hülse gemessen werden. Eine derartige Hülse könnte am Drucksensor befestigt sein und blasenfrei mit vorzugsweise entgastem Wasser gefüllt sein. Die Hülse könnte aus Metall oder Kunststoff ausgebildet sein. Bei der Messung überträgt die Wassersäule in der Hülse den Druck im Porenwasser des Baustoffs, beispielsweise Beton, auf die Sensorfläche. Wird die Spitze des Drucksensors bei der Umverteilung des Wassers belüftet, so bricht der gemessene Druck zusammen. Liegt in diesem Fall die Sensorspitze in einem Inselbildungsbereich mit noch vollständig wassergefüllten, d.h. noch nicht belüfteten Bereichen, so erfolgt der Zusammenbruch des Kapillardrucks dort später als in anderen Bereichen. Dabei steigt der Druck im Bereich der Sensorspitze zumeist weiter an.

Weiterhin im Hinblick auf eine sichere Bestimmung des Lufteintrittspunkts könnten zur Messung des Kapillardrucks mehrere Drucksensoren oder Hülsen in den Baustoff eingebracht oder einbetoniert werden. Damit ist eine Messung des Kapillardrucks an unterschiedlichen Stellen im Baustoff ermöglicht.

Für die Ergebnisbeurteilung der Messung könnten in vorteilhafter Weise weitere Kennwerte erfasst werden. Dabei könnte beispielsweise zusätzlich die Setzung des Baustoffs erfasst werden. Alternativ oder zusätzlich hierzu könnte das Schwindmaß des Baustoffs und/oder die Temperatur des Baustoffs erfasst bzw. gemessen werden. Dabei könnte die Temperatur in verschiedenen Tiefen im Baustoff gemessen werden, um einen Temperaturverlauf in Abhängigkeit von der Tiefe im Baustoff zu ermitteln.

In weiter vorteilhafter Weise könnte zusätzlich die relative Luftfeuchte und/oder die Lufttemperatur in der Umgebung des Baustoffs gemessen werden.

Je nach Anwendungsfall könnten die Setzung und/oder das Schwindmaß und/oder die Temperatur und/oder die Luftfeuchte und/oder die Lufttemperatur kontinuierlich oder nach vorgebbaren Zeitabständen oder an vorgebbaren Zeitpunkten innerhalb eines vorgebbaren Zeitintervalls gemessen werden.

Erfindungsgemäß wird unter Berücksichtigung einer signifikanten Veränderung der Leitfähigkeit ein Schwellwert für den Kapillardruck bestimmt. Mit anderen Worten lässt sich beispielsweise im Laborversuch bei gleichzeitiger Messung von Kapillardruck und Leitfähigkeit unter vorgegebenen Klimabedingungen der Kapillardruck zum Zeitpunkt der Porenbelüftung bestimmen und somit ein Schwellwert unterhalb des Lufteintrittspunkts festlegen, ab dem eine Nachbehandlung zur Rissvermeidung infolge kapillaren Schwindens erforderlich wird. Nach einer Schwellwertermittlung im Labor reicht auf der Baustelle die Messung des Kapillardrucks aus. Die Messung der Leitfähigkeit im Baustoff ist dann nicht mehr erforderlich. Diese Vorgehensweise ist aufgrund des geringen Mess- und Auswerteaufwands, der geringeren Schädigung der Baustoff- oder Betonoberfläche durch die Sensoren, der leichteren Demontage der Sensoren sowie der geringen Sensorkosten vorteilhaft. Im Labor befüllte, einsatzfertige Kapillardruck-Sensoren ermöglichen eine schnelle und einfache Handhabung auf der Baustelle. Um Fehlmessungen vorzubeugen, sollten jeweils mindestens zwei Sensoren verwendet werden.

In besonders vorteilhafter Weise könnte das Erreichen des Schwellwerts für den Kapillardruck durch ein optisches und/oder akustisches Signal angezeigt werden. Bei oder nach Erreichen des Schwellwerts für den Kapillardruck erfolgt erfindungsgemäß eine Nachbehandlung des Baustoffs. Durch das obige optische und/oder akustische Signal könnte auf die erforderliche Nachbehandlung aufmerksam gemacht werden.

Als besonders vorteilhafte Nachbehandlung hat sich eine Folienabdeckung des Baustoffs oder ein Aufbringen eines Curing-Mittels auf den Baustoff gezeigt.

Alternativ oder zusätzlich hierzu könnte zur Nachbehandlung ein Verneblungs- und/oder Beregnungssystem verwendet werden. Eine automatisierte Nachbehandlung könnte über eine an einen oder mehrere Drucksensoren angeschlossene Steuerelektronik erfolgen.

Mit der Kapillardruckmessung wird die das kapillare Schwinden bestimmende Größe - der Kapillardruck - direkt bestimmt und kann für die Betrachtung der Rissgefährdung herangezogen werden. Eine genäherte, indirekte Beurteilung über die Verdunstungsrate ist nicht nötig. Durch den mittels Leitfähigkeitsmessung ermittelten Lufteintrittspunkt erhält man einen material- und/oder mischungsspezifischen Kapillardruck, ab dem eine erhöhte Rissgefährdung vorliegt. Unter Berücksichtigung eines Sicherheitsabstands lässt sich in situ der exakte Zeitpunkt bestimmen, ab dem eine Nachbehandlung erforderlich ist.

Letztendlich wird mit dem erfindungsgemäßen Verfahren ein In-Situ-Messverfahren zur Vermeidung von Baustoff- oder Betonschäden infolge plastischen Schwindens bereitgestellt.

Des Weiteren wird nachfolgend eine Vorrichtung zur Untersuchung eines Materialverhaltens in einem Baustoff, insbesondere in Beton, bechrieben. Danach sind mit dieser Vorrichtung einerseits der Kapillardruck und andererseits die elektrische Leitfähigkeit im Baustoff messbar. Hinsichtlich der Vorteile der Vorrichtung wird zur Vermeidung von Wiederholungen auf die entsprechenden Passagen bei der Beschreibung des voranstehenden Verfahrens zur Untersuchung eines Materialverhaltens in einem Baustoff verwiesen, wo auf die Kombination der Kapillardruck- und Leitfähigkeitsmessung bereits eingegangen wird.

Im Konkreten könnte der Kapillardruck mittels eines eine Sensorfläche aufweisenden Drucksensors messbar sein. An einen derartigen Drucksensor könnte ein Fühler angekoppelt sein, der vorzugsweise eine mit Wasser gefüllte Hülse ist. Die Hülse könnte in besonders einfacher Weise aus Kunststoff oder Metall ausgebildet sein und vorzugsweise eine konische Form aufweisen. Dies vereinfacht die Entnahme der Hülse aus dem gefestigten Baustoff oder Beton am Ende der Messung. Das hierdurch im Baustoff oder Beton verbleibende Loch lässt sich auf einfache Weise befüllen und zum Gebrauch des mit dem Baustoff hergestellten Bauwerks verschließen.

Im Konkreten könnte die Hülse eine wassergefüllte Pipettenspitze aus transparentem Kunststoff sein. Die Drucksensoren halten ihre Position und Einbauhöhe aufgrund der Mantelreibung der Pipettenspitze. Bei einer konischen Form könnte die Spitze nach der Messung problemlos aus dem Baustoff entfernt werden.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines Ausführungsbeispiels der erfindungsgemäßen Lehre anhand der Zeichnung zu verweisen. In Verbindung mit der Erläuterung des bevorzugten Ausführungsbeispiels der erfindungsgemäßen Lehre anhand der Zeichnung werden auch im Allgemeinen bevorzugte Ausgestaltungen und Weiterbildungen der Lehre erläutert. In der Zeichnung zeigen

Fig. 1          in einer schematischen Seitenansicht ein Ausführungsbeispiel einer für das erfindungsgemäßeVerfahren geeigneten Vorrichtung zur Untersuchung eines Materialverhaltens in einem Baustoff,

Fig. 2 (a)     in einem Diagramm einen typischen Kapillardruckverlauf in Abhängigkeit von der Zeit und

Fig. 2 (b)     in einem Diagramm einen typischen Verlauf des elektrischen Widerstands in Abhängigkeit von der Zeit.

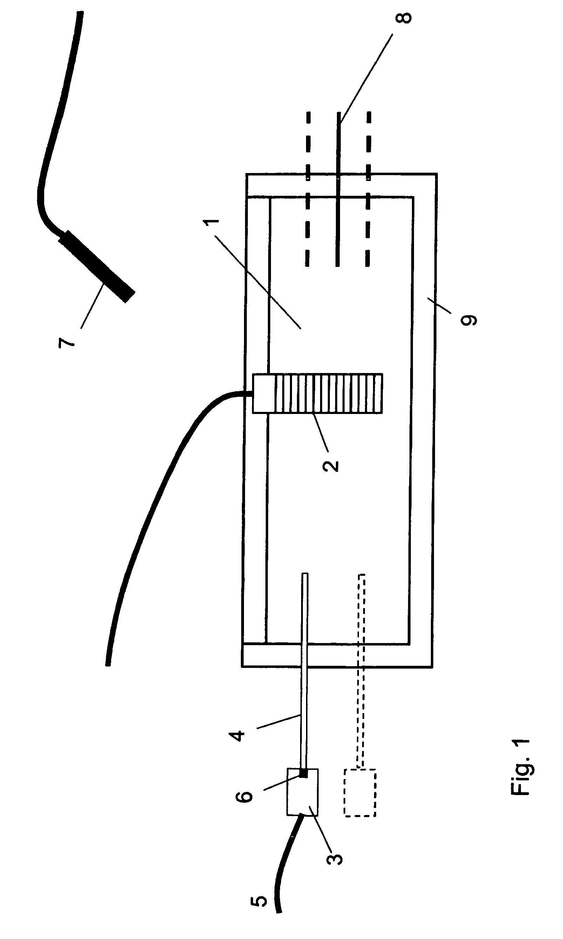

Fig. 1 zeigt in einer schematischen Seitenansicht ein Ausführungsbeispiel einer für das erfindungsgemäße Verfahren geeigneten Vorrichtung zur Untersuchung eines Materialverhaltens in einem Baustoff 1, wobei als Baustoff 1 hier Beton verwendet ist. Mit der Vorrichtung sind einerseits der Kapillardruck und andererseits die elektrische Leitfähigkeit im Baustoff 1 messbar. Hierdurch ist der Lufteintrittspunkt bei der Porenbelüftung sicher identifizierbar.

Der Kapillardruck und die Leitfähigkeit werden gleichzeitig innerhalb eines vorgebbaren Zeitintervalls ab dem Einbau des Baustoffs gemessen. Dabei findet eine kontinuierliche Messung des Kapillardrucks und der Leitfähigkeit statt.

Die Leitfähigkeit wird in unterschiedlichen Tiefen in dem Baustoff 1 gemessen, wozu eine tiefengestaffelte Leitfähigkeitssonde 2 verwendet wird.

Der Kapillardruck wird mittels eines Drucksensors 3 mit einer in den Baustoff 1 eingebrachten oder einbetonierten Hülse 4 gemessen. Der Kapillardruck ist in unterschiedlichen Tiefen in dem Baustoff 1 messbar, wie dies durch die gestrichelte Darstellung des Drucksensors 3 in einem tieferen Bereich des Baustoffs 1 gezeigt ist.

Zur Messung des Kapillardrucks können mehrere Drucksensoren 3 oder Hülsen 4 in den Baustoff 1 eingebracht oder einbetoniert werden. Bei dem hier gezeigten Ausführungsbeispiel handelt es sich um einen Laborversuch, wobei eine Messung des Kapillardrucks auch direkt in dem zu errichtenden Bauwerk auf einer Baustelle erfolgen kann.

Der Drucksensor 3 ist mit einer Steuerelektronik 5 verbunden, die eine automatisierte Nachbehandlung des Baustoffs 1 beginnen oder steuern kann.

Der Drucksensor 3 weist eine Sensorfläche 6 auf, auf die der Druck im Porenwasser über das in der Hülse 4 eingebrachte Wasser übertragen wird.

Bei dem hier gezeigten Aufbau ist des Weiteren ein Sensor 7 für die relative Luftfeuchte und Lufttemperatur vorgesehen. Des Weiteren sind Temperatursensoren 8 in unterschiedlichen Tiefen im Baustoff 1 eingebracht. Der Baustoff 1 ist in einem Behälter 9 angeordnet.

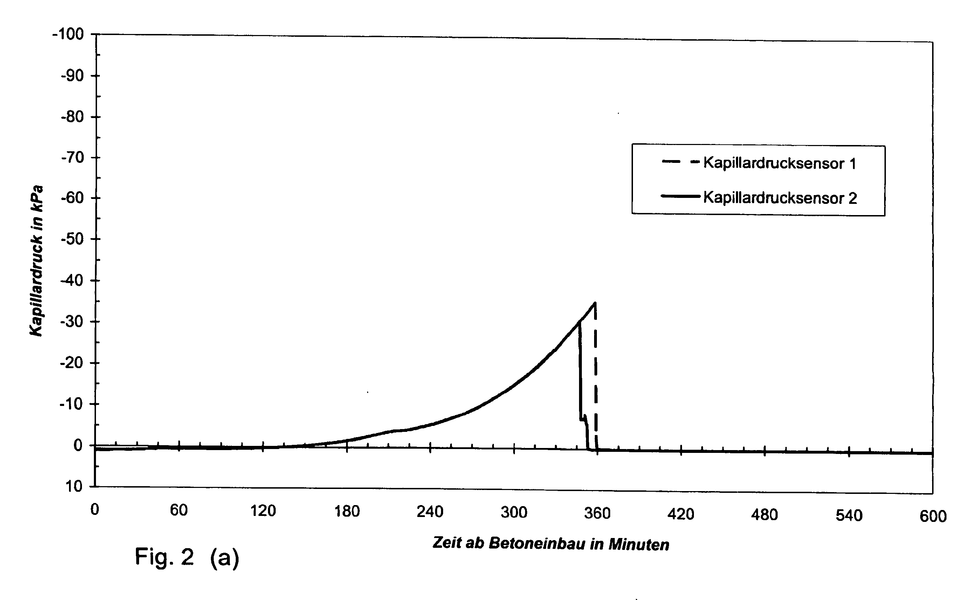

Fig. 2(a) zeigt einen typischen Kapillardruckverlauf in Abhängigkeit von der Zeit ab dem Betoneinbau. Der Aufbau des Drucks beginnt hier etwa zwei Stunden nach dem Einbau. Nach etwa sechs Stunden erfolgt bei etwa 35 kPa der Zusammenbruch des Kapillardrucks.

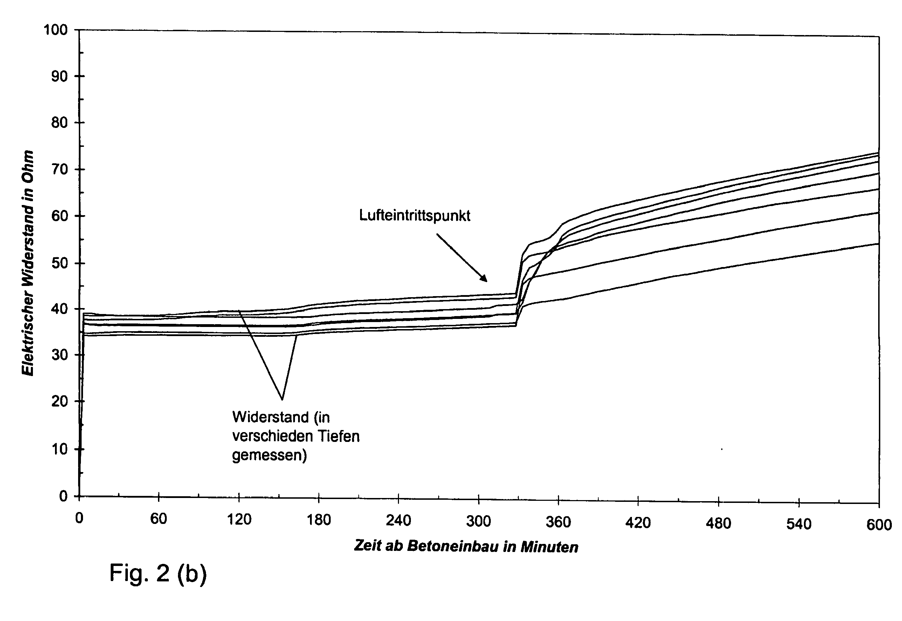

Fig. 2 (b) zeigt in einem Diagramm einen typischen Verlauf des elektrischen Widerstands in Abhängigkeit von der Zeit ab Einbau. Das Diagramm zeigt sieben Messlinien, die eine Messung in unterschiedlichen Tiefen im Baustoff 1 dokumentieren. Am Lufteintrittspunkt ist eine signifikante Änderung der Leitfähigkeit des Baustoffs 1 und damit des elektrischen Widerstands feststellbar.

Hinsichtlich weiterer vorteilhafter Ausgestaltungen des erfindungsgemäßen Verfahrens wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel lediglich zur Erörterung der beanspruchten Lehre dient, diese jedoch nicht einschränkt.

**Patentansprüche**

1. Verfahren zur Untersuchung eines Materialverhaltens in einem Baustoff (1), insbesondere in Beton, bei dem einerseits der Kapillardruck und andererseits die elektrische Leitfähigkeit im Baustoff (1) gemessen werden, wobei unter Berücksichtigung einer signifikanten Veränderung der Leitfähigkeit ein Schwellwert für den Kapillardruck bestimmt wird, ab dem Einbau des Baustoffs (1) der Kapillardruck gemessen wird und bei oder nach Erreichen des Schwellwerts für den Kapillardruck eine Nachbehandlung des Baustoffs (1) erfolgt.

2. Verfahren nach Anspruch 1, **dadurch gekennzeichnet, dass** der Kapillardruck kontinuierlich oder nach vorgebbaren Zeitabständen oder an vorgebbaren Zeitpunkten innerhalb eines vorgebbaren Zeitintervalls gemessen wird.

3. Verfahren nach Anspruch 1 oder 2, **dadurch gekennzeichnet, dass** die Leitfähigkeit kontinuierlich oder nach vorgebbaren Zeitabständen oder an vorgebbaren Zeitpunkten innerhalb eines vorgebbaren Zeitintervalls gemessen wird.

4. Verfahren nach einem der Ansprüche 1 bis 3, **dadurch gekennzeichnet, dass** der Kapillardruck und die Leitfähigkeit gleichzeitig gemessen werden.

5. Verfahren nach einem der Ansprüche 1 bis 4, **dadurch gekennzeichnet, dass** die Leitfähigkeit in unterschiedlichen Tiefen in dem Baustoff (1) gemessen wird und/oder dass zur Messung der Leitfähigkeit eine tiefengestaffelte Leitfähigkeitssonde (2) verwendet wird.

6. Verfahren nach einem der Ansprüche 1 bis 5, **dadurch gekennzeichnet, dass** der Kapillardruck mittels eines Drucksensors (3) mit einer in den Baustoff (1) eingebrachten oder einbetonierten Hülse (4) gemessen wird und/oder dass der Kapillardruck in unterschiedlichen Tiefen in dem Baustoff (1) gemessen wird und/oder dass zur Messung des Kapillardrucks mehrere Drucksensoren (3) oder Hülsen (4) in den Baustoff (1) eingebracht oder einbetoniert werden.

7. Verfahren nach einem der Ansprüche 1 bis 6, **dadurch gekennzeichnet, dass** zusätzlich die Setzung des Baustoffs (1) erfasst wird und/oder dass zusätzlich das Schwindmaß des Baustoffs (1) erfasst wird.

8. Verfahren nach einem der Ansprüche 1 bis 7, **dadurch gekennzeichnet, dass** zusätzlich die Temperatur des Baustoffs (1) gemessen wird und/oder dass die Temperatur in verschiedenen Tiefen im Baustoff (1) gemessen wird.

9. Verfahren nach einem der Ansprüche 1 bis 8, **dadurch gekennzeichnet, dass** zusätzlich die relative Luftfeuchte in der Umgebung des Baustoffs (1) gemessen wird und/oder dass zusätzlich die Lufttemperatur in der Umgebung des Baustoffs (1) gemessen wird.

10. Verfahren nach einem der Ansprüche 7 bis 9, **dadurch gekennzeichnet, dass** die Setzung und/oder das Schwindmaß und/oder die Temperatur und/oder die Luftfeuchte und/oder die Lufttemperatur kontinuierlich oder nach vorgebbaren Zeitabständen oder an vorgebbaren Zeitpunkten innerhalb eines vorgebbaren Zeitintervalls gemessen werden oder wird.

11. Verfahren nach einem der Ansprüche 1 bis 10, **dadurch gekennzeichnet, dass** das Erreichen des Schwellwerts für den Kapillardruck durch ein optisches und/oder akustisches Signal angezeigt wird.

12. Verfahren nach einem der Ansprüche 1 bis 11, **dadurch gekennzeichnet, dass** als Nachbehandlung eine Folienabdeckung des Baustoffs (1) oder ein Aufbringen eines Curing-Mittels auf den Baustoff (1) durchgeführt wird und/oder dass zur Nachbehandlung ein Verneblungs- und/oder Beregnungssystem verwendet wird und/oder dass eine automatisierte Nachbehandlung über eine an einen oder mehrere Drucksensoren (3) angeschlossene Steuerelektronik (5) erfolgt.

**Claims**

1. A method for inspecting the material behaviour in a building material (1), in particular in concrete, in which on the one hand the capillary pressure and on the other hand the electrical conductivity in the building material (1) are measured, wherein, allowing for a significant change in the conductivity a threshold value for the capillary pressure is determined, from the installation of the building material (1) the capillary pressure is measured and at or after reaching the threshold value for the capillary pressure an after-treatment of the building material (1) is effected.

2. A method according to claim 1, **characterised in that** the capillary pressure is measured continuously or after predeterminable intervals or at predeterminable times within a predeterminable time period.

3. A method according to claim 1 or 2, **characterised in that** the conductivity is measured continuously or after predeterminable intervals or at predeterminable times within a predeterminable time period.

4. A method according to any one of claims 1 to 3, **characterised in that** the capillary pressure and the conductivity are measured simultaneously.

5. A method according to any one of claims 1 to 4, **characterised in that** the conductivity is measured in at different depths in the building material (1) and/or a depth-graduated conductivity probe (2) is used to measure the conductivity.

6. A method according to any one of claims 1 to 5, **characterised in that** the capillary pressure is measured by means of a pressure sensor (3) with a tube (4) introduced into or concreted into the building material (1) and/or the capillary pressure is measured at different depths in the building material (1) and/or in order to measure the capillary pressure a plurality of pressure sensors (3) or tubes (4) are introduced into or cemented into the building material (1).

7. A method according to any one of claims 1 to 6, **characterised in that** in addition the settling of the building material (1) is detected and/or the shrinkage of the building material (1) is detected.

8. A method according to any one of claims 1 to 7, **characterised in that** in addition the temperature of the building material (1) is measured and/or the temperature at different depths in the building material (1) is measured.

9. A method according to any one of claims 1 to 8, **characterised in that** in addition the relative humidity in the area surrounding the building material (1) is measured and/or in addition the air temperature in the area surrounding the building material (1) is measured.

10. A method according to any one of claims 7 to 9, **characterised in that** the settling and/or the shrinkage and/or the temperature and/or the air humidity and/or the air temperature are/is measured continuously or after predeterminable intervals or at predeterminable times within a predeterminable period.

11. A method according to any one of claims 1 to 10, **characterised in that** reaching the threshold value for the capillary pressure is indicated by an optical and/or acoustic signal.

12. A method according to any one of claims 1 to 11, **characterised in that** as after-treatment the building material (1) is covered with film or a curing agent is applied to the building material (1) and/or a misting and/or sprinkler system is used for the after-treatment and/or an automated after-treatment is effected by way of control electronics (5) connected to one or more pressure sensors (3).

**Revendications**

1. Procédé destiné à l'examen du comportement d'un matériau dans un matériau de construction (1), en particulier dans le béton, dans lequel, d'une part, la pression capillaire, et d'autre part, la conductibilité électrique dans le matériau de construction (1) sont mesurées,
étant entendu qu'une valeur de seuil pour la pression capillaire est déterminée en prenant en considération une modification significative de la conductibilité, la pression capillaire est mesurée à partir de l'installation du matériau de construction (1) et un traitement subséquent du matériau de construction (1) est réalisé lorsque la pression capillaire a atteint ou dépassé la valeur de seuil.

2. Procédé selon la revendication 1, **caractérisé en ce que** la pression capillaire est mesurée de façon continue ou après des intervalles de temps pouvant être prédéterminés ou à des moments pouvant être prédéterminés à l'intérieur d'un intervalle de temps pouvant être prédéterminé.

3. Procédé selon la revendication 1 ou 2, **caractérisé en ce que** la conductibilité est mesurée de façon continue ou après des intervalles de temps pouvant être prédéterminés ou à des moments pouvant être prédéterminés à l'intérieur d'un intervalle de temps pouvant être prédéterminé.

4. Procédé selon l'une des revendications 1 à 3, **caractérisé en ce que** la pression capillaire et la conductibilité sont mesurées en même temps.

5. Procédé selon l'une des revendications 1 à 4, **caractérisé en ce que** la conductibilité est mesurée à des profondeurs différentes dans le matériau de construction (1) et/ou **en ce qu'**une sonde de conductibilité (2) ayant une graduation de la profondeur est utilisée pour mesurer la conductibilité.

6. Procédé selon l'une des revendications 1 à 5, **caractérisé en ce que** la pression capillaire est mesurée au moyen d'un capteur de pression (3) avec un manchon (4) inséré ou noyé dans du béton dans le matériau de construction (1) et/ou **en ce que** la pression capillaire est mesurée à des profondeurs différentes dans le matériau de construction (1) et/ou **en ce que** plusieurs capteurs de pression (3) ou manchons (4) sont insérés ou noyés dans du béton dans le matériau de construction (1) pour mesurer la pression capillaire.

7. Procédé selon l'une des revendications 1 à 6, **caractérisé en ce qu'**en complément, le tassement du matériau de construction (1) est détecté et/ou **en ce qu'**en complément, le coefficient de retrait du matériau de construction (1) est détecté.

8. Procédé selon l'une des revendications 1 à 7, **caractérisé en ce qu'**en complément, la température du matériau de construction (1) est mesurée et/ou **en ce que** la température est mesurée à des profondeurs différentes dans le matériau de construction (1).

9. Procédé selon l'une des revendications 1 à 8, **caractérisé en ce qu'**en complément, l'humidité relative de l'air dans l'environnement du matériau de construction (1) est mesurée et/ou **en ce qu'**en complément, la température ambiante dans l'environnement du matériau de construction (1) est mesurée.

10. Procédé selon l'une des revendications 7 à 9, **caractérisé en ce que** le tassement et/ou le coefficient de retrait et/ou la température et/ou l'humidité de l'air et/ou la température ambiante sont mesurés de façon continue ou après des intervalles de temps pouvant être prédéterminés ou à des moments pouvant être prédéterminés à l'intérieur d'un intervalle de temps pouvant être prédéterminé.

11. Procédé selon l'une des revendications 1 à 10, **caractérisé en ce que** l'arrivée de la pression capillaire à la valeur de seuil est indiquée par un signal visuel et/ou acoustique.

12. Procédé selon l'une des revendications 1 à 11, **caractérisé en ce qu'**en tant que traitement subséquent, un recouvrement du matériau de construction (1) par une feuille plastique ou une application d'un moyen de cure sur le matériau de construction (1) est exécuté(e) et/ou en ce qu'aux fins du traitement subséquent, un système de nébulisation et/ou d'aspersion est utilisé et/ou **en ce qu'**un traitement subséquent automatisé est réalisé par le biais d'un dispositif électronique de commande (5) relié à un ou plusieurs capteurs de pression (3).

Fig. 1

Fig. 2 (a)

Fig. 2 (b)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040153270 A1